(19) Europäisches Patentamt
European Patent Office
Office européen des brevets

(11) Veröffentlichungsnummer: **0 088 259**
**A2**

(12) # EUROPÄISCHE PATENTANMELDUNG

(21) Anmeldenummer: **83101501.1**

(22) Anmeldetag: **17.02.83**

(51) Int. Cl.³: **C 07 D 403/04**, A 01 N 43/64
// C07D207/36

(30) Priorität: **17.02.82 DE 3205682**

(43) Veröffentlichungstag der Anmeldung: **14.09.83**
**Patentblatt 83/37**

(84) Benannte Vertragsstaaten: **BE DE FR GB IT NL**

(71) Anmelder: **Consortium für elektrochemische Industrie GmbH, Zielstattstrasse 20, D-8000 München 70 (DE)**

(72) Erfinder: **Schilling, Bernd, Dr. Dipl.-Chem.,**
**Willibaldstrasse 12b, D-8000 München 21 (DE)**
Erfinder: **Kinzel, Peter, Ing.-grad.,**
**Jägerkampstrasse 21a, D-8152 Westerham/Feldkirchen (DE)**
Erfinder: **Reutter, Anneliese, Dipl.-Ing.,**
**Ottobrunnerstrasse 13, D-8025 Unterhaching (DE)**

(54) **Azolyl-pyrrolidinone, Verfahren zu deren Herstellung und ihre Verwendung als Fungizide.**

(57) Die Erfindung betrifft Azolyl-pyrrolidinone der Formel

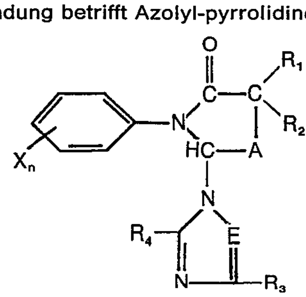

sowie deren Säureadditionssalze und Metallsalz-Komplexe, wobei

$R_1$ und $R_2$ gleiche oder verschiedene Alkylreste mit 1 bis 3 Kohlenstoffatomen bedeuten,

$R_3$ und $R_4$ gleiche oder verschiedene Substituenten sind mit der Bedeutung Wasserstoff, Alkyl mit 1 bis 4 Kohlenstoffatomen und Halogen,

A für eine Ketogruppe, für ein funktionelles Derivat einer Ketogruppe und für eine alkoholische Gruppe der Formel = CH-OH steht,

E ein Stickstoffatom oder die ⩾CH-Gruppe bedeutet

X für Substituenten steht, die ausgewählt sind aus der Gruppe der Halogene, der Cyano- und der Nitrogruppen, der Alkylgruppen mit 1 bis 3 Kohlenstoffatomen, der substituierten Alkylgruppen mit 1 bis 3 Kohlenstoffatomen, der Cycloalkylgrupen mit 3 bis 6 Kohlenstoffatomen, der Alkoxygruppe mit 1 bis 3 Kohlenstoffatomen, der Alkylthiogruppen mit 1 bis 3 Kohlenstoffatomen, der Alkylsulfonylgruppen mit 1 bis 3 Kohlenstoffatomen, der Acyloxygruppen mit 1 bis 3 Kohlenstoffatomen und der, ggf. substituierten Phenyl- und Pheoxygruppen und

n 0 oder eine ganze Zahl von 1 bis 5 ist.

Die erfindungsgemäßen Verbindungen weisen fungizide Eigenschaften auf.

EP 0 088 259 A2

C o n s o r t i u m
für elektrochemische Industrie
GmbH

München, de
LC-PAT/Dr.R

0088259

Co 8107

# Azolyl-pyrrolidinone, Verfahren zu deren Herstellung und ihre Verwendung als Fungizide

Gegenstand der Erfindung sind Verbindungen der allgemeinen Formel

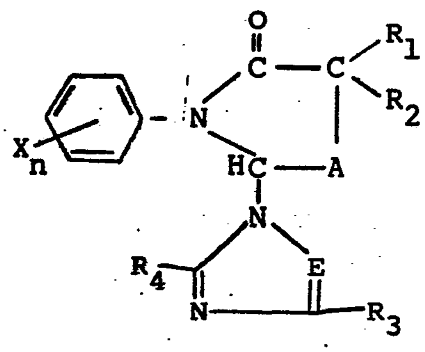

sowie deren Säureadditionssalze und Metallsalz-Komplexe, wobei

$R_1$ und $R_2$ gleiche oder verschiedene Alkylreste mit 1 bis 3 Kohlenstoffatomen bedeuten,

$R_3$ und $R_4$ gleiche oder verschiedene Substituenten sind mit der Bedeutung Wasserstoff, Alkyl mit 1 bis 4 Kohlenstoffatomen und Halogen,

A für eine Ketogruppe, für ein funktionelles Derivat einer Ketogruppe und für eine alkoholische Gruppe der Formel = CH - OH steht,

E ein Stickstoffatom oder die $\geqslant$CH-Gruppe bedeutet

X für Substituenten steht, die ausgewählt sind aus der Gruppe der Halogene, der Cyano- und der Nitrogruppen, der Alkylgruppen mit 1 bis 3 Kohlenstoffatomen, der substituierten Alkylgruppen mit 1 bis 3 Kohlenstoffatomen, der Cycloalkylgruppen mit 3 bis 6 Kohlenstoffatomen, der Alkoxygruppen mit 1 bis 3 Kohlenstoffatomen, der Alkylthiogruppen mit 1 bis 3 Kohlenstoffatomen, der Alkylsulfonylgruppen

mit 1 bis 3 Kohlenstoffatomen, der Acyloxygruppen mit 1 bis 3 Kohlenstoffatomen und der, ggf. substituierten Phenyl- und Phenoxygruppen und

n 0 oder eine ganze Zahl von 1 bis 5 ist.

$R_1$ und $R_2$ sind vorzugsweise Methylgruppen. Weitere Beispiele sind die Ethyl-, die n-Propyl- und die Iso-propylgruppe.

$R_3$ und $R_4$ stehen vorzugsweise für Wasserstoff. Weitere Beispiele sind Alkylreste, wie Methyl, Ethyl, n-Propyl-, Iso-propyl sowie die Butylreste. Als Halogenreste kommen der Fluor-, der Chlor- und der Bromrest, insbesondere der Chlorrest, in Betracht.

Ein Beispiel für ein funktionelles Derivat einer Ketogruppe (entsprechend A) ist insbesondere die Oximgruppe.

Beispiele für Substituenten X sind Halogene, wie Fluor, Chlor, Brom, Jod, insbesondere Chlor; Alkylgruppen, wie die Methyl-, die Ethyl-, die n-Propyl- und die Iso-propylgruppe; substituierte Alkylgruppen mit 1 bis 3 Kohlenstoffatomen, wie der Chlormethylrest, der Trifluormethylrest, der 3.3.3-Trifluorpropylrest, der Hydroxymethylrest; Cycloalkylgruppen, wie der Cyclopropan-, der Cyclobutan-, der Cyclopentan- und der Cyclohexanrest; Alkoxygruppen, wie der Methoxy-, Ethoxy- und n-Propyloxyrest; Acyloxygruppen, wie der Formyl-, der Acetyl- und der Propionylrest. Beispiele für substituierte Phenyl- und Phenoxygruppen sind der p-Chlorphenyl-, der p-Chlorphenoxy-, der 2.4-Dichlorphenyl-, der 2.4-Dichlorphenoxy-, der p-Methylphenyl-, der p-Methylphenoxy-, der p-Methoxyphenyl-, der p-Nitrophenyl-, der m-Trifluormethylphenyl-, der 2-Methyl-4-chlorphenylrest u.a.

Vorzugsweise bedeutet n 0 oder eine ganze Zahl von 1 bis 3, insbesondere 0, 1 oder 2.

Die durch das Symbol $X_n$ bezeichneten Substituenten können sich im Fall der Monosubstitution in Ortho-, Metha- oder Parastellung zum Stickstoffatom befinden. Im Fall der Disubstitution ist die 2.4-, die 2.6-, die 3.5- und insbesondere die 2.4-Substitution bevorzugt. Ein Beispiel für Trisubstitution ist insbesondere die 2.4.6-Substitution.

Spezielle Beispiele für erfindungsgemäße Verbindungen sind:

N-Phenyl-3.3-dimethyl-5-(1.2.4-triazol-1-yl)-pyrrolidindion(2.4)
N-(4-Chlorphenyl)-3.3-dimethyl-5-(1.2.4-triazol-1-yl)-pyrrolidindion(2.4)
N-(4-Fluorphenyl)-3.3-dimethyl-5-(1.2.4-triazol-1-yl)-pyrrolidindion(2.4)
N-(4-Bromphenyl)-3.3-dimethyl-5-(1.2.4-triazol-1-yl)-pyrrolidindion(2.4)
N-(4-Nitrophenyl)-3.3-dimethyl-5-(1.2.4-triazol-1-yl)-pyrrolidindion(2.4)
N-(4-Cyanophenyl)-3.3-dimethyl-5-(1.2.4-triazol-1-yl)-pyrrolidindion(2.4)
N-(4-Methoxyphenyl)-3.3-dimethyl-5-(1.2.4-triazol-1-yl)-pyrrolidindion(2.4)
N-(4-Phenoxyphenyl)-3.3-dimethyl-5-(1.2.4-triazol-1-yl)-pyrrolidindion(2.4)
N-[4-(4'-Chlorphenoxy)-phenyl]-3.3-dimethyl-5-(1.2.4-triazol-1-yl)-pyrrolidindion(2.4)
N-[4-(2'.4'-Dichlorphenoxy)-phenyl]-3.3-dimethyl-5-(1.2.4-triazol-1-yl)-pyrrolidindion(2.4)
N-(4-Methylphenyl)-3.3-dimethyl-5-(1.2.4-triazol-1-yl)-pyrrolidindion(2.4)
N-(4-Ethylphenyl)-3.3-dimethyl-5-(1.2.4-triazol-1-yl)-pyrrolidindion(2.4)
N-(4-n-Butylphenyl)-3.3-dimethyl-5-(1.2.4-triazol-1-yl)-pyrrolidindion(2.4)
N-(3-Chlorphenyl)-3.3-dimethyl-5-(1.2.4-triazol-1-yl)-pyrrolidindion(2.4)

N-(2-Chlorphenyl)-3.3-dimethyl-5-(1.2.4-triazol-1-yl)-pyrrolidindion(2.4)

N-(2-Methylphenyl)-3.3-dimethyl-5-(1.2.4-triazol-1-yl)-pyrrolidindion(2.4)

N-(2-Methoxyphenyl)-3.3-dimethyl-5-(1.2.4-triazol-1-yl)-pyrrolidindion(2.4)

N-(2.4-Difluorphenyl)-3.3-dimethyl-5-(1.2.4-triazol-1-yl)-pyrrolidindion(2.4)

N-(4-Chlor-2-Methyl-phenyl)-3.3-dimethyl-5-(1.2.4-triazol-1-yl)-pyrrolidindion(2.4)

N-(3-Fluor-4-Chlor-phenyl)-3.3-dimethyl-5-(1.2.4-triazol-1-yl)-pyrrolidindion(2.4)

N-(2.6-Dichlor-phenyl)-3.3-dimethyl-5-(1.2.4-triazol-1-yl)-pyrrolidindion(2.4)

N-(2.4.6-Trichlorphenyl)-3.3-dimethyl-5-(1.2.4-triazol-1-yl)-pyrrolidindion(2.4)

N-(3.5-Dichlorphenyl)-3.3-dimethyl-5-(1.2.4-triazol-1-yl)-pyrrolidindion(2.4)

N-(3-Fluorphenyl)-3.3-dimethyl-5-(1.2.4-triazol-1-yl)-pyrrolidindion(2.4)

N-(2.6-Dimethylphenyl)-3.3-dimethyl-5-(1.2.4-triazol-1-yl)-pyrrolidindion(2.4)

N-(3-Trifluormethyl-phenyl)-3.3-dimethyl-5-(1.2.4-triazol-1-yl)-pyrrolidindion(2.4)

N-(4-Methylthiophenyl)-3.3-dimethyl-5-(1.2.4-triazol-1-yl)-pyrrolidindion(2.4)

N-4-Methylsulfonyl-phenyl)-3.3-dimethyl-5-(1.2.4-triazol-1-yl)-pyrrolidindion(2.4)

N-(4-Carboxymethyl-phenyl)-3.3-dimethyl-5-(1.2.4-triazol-1-yl)-pyrrolidindion(2.4)

N-(4-Chlorphenyl)-3.3-diethyl-5-(1.2.4-triazol-1-yl)-pyrrolidindion(2.4)

N-(2.4-Dichlorphenyl)-3.3-diethyl-5-(1.2.4-triazol-1-yl)-pyrrolidindion(2.4)

N-(2.4-Dichlorphenyl)-3.3-dipropyl-5-(1.2.4-triazol-1-yl)-pyrrolidindion(2.4)

N-(2.4-Dichlorphenyl)-3-methyl-3-ethyl-5-(1.2.4-triazol-1-yl)-pyrrolidindion(2.4)

N-(2.4-Dichlorphenyl)-3.3-dimethyl-5-(imidazol-1-yl)-pyrrolidindion(2.4)

N-(4-Chlorphenyl)-3.3-dimethyl-5-(imidazol-1-yl)-pyrrolidindion(2.4)

N-(4-Bromphenyl)-3.3-dimethyl-5-(imidazol-1-yl)-pyrrolidindion(2.4)

N-(4-Fluorphenyl)-3.3-dimethyl-5-(imidazol-1-yl)-pyrrolidindion(2.4)

N-(4-Nitrophenyl)-3.3-dimethyl-5-(imidazol-1-yl)-pyrrolidindion(2.4)

N-(4-Nitrophenyl)-3.3-dimethyl-5-(imidazol-1-yl)-pyrrolidindion(2.4)-hydrochlorid

N-(2.4-Dichlorphenyl)-3.3-dimethyl-5-(imidazol-1-yl)-pyrrolidindion(2.4)-hydrochlorid

N-(4-Chlorphenyl)-3.3-dimethyl-5-(imidazol-1-yl)-pyrrolidindion(2.4)-hydrochlorid

N-(3.5-Dichlorphenyl)-3.3-dimethyl-5-(imidazol-1-yl)-pyrrolidindion(2.4)-hydrochlorid

N-(2.4-Dichlorphenyl)-3.3-dimethyl-5-(imidazol-1-yl)-pyrrolidindion(2.4)-hydrogensulfat

N-(2.4-Dichlorphenyl)-3.3-dimethyl-5-(imidazol-1-yl)-pyrrolidindion(2.4)-cuprichlorid

N-(2.4-Dichlorphenyl)-3.3-dimethyl-5-(imidazol-1-yl)-pyrrolidindion(2.4)-Zink-chlorid

N-(2.4-Dichlorphenyl)-3.3-dimethyl-5-(imidazol-1-yl)-pyrrolidindion(2.4)-hydrogennitrat

N-(2.4-Dichlorphenyl)-3.3-dimethyl-5-(imidazol-1-yl)-pyrrolidindion(2.4)-tosylat

N-(2.4-Dichlorphenyl)-3.3-dimethyl-5-(triazol-1-yl)-pyrrolidindion(2.4)-hydrochlorid

N-(4-Chlorphenyl)-3.3-dimethyl-5-(triazol-1-yl)-pyrrolidindion(2.4)-hydrochlorid

N-(4-Chlorphenyl)-3.3-dimethyl-5-(triazol-1-yl)-pyrrolidindion(2.4)-hydrogensulfat

N-(4-Chlorphenyl)-3.3-dimethyl-5-(triazol-1-yl)-pyrrolidindion(2.4)- cuprichlorid

N-(4-Chlorphenyl)-3.3-dimethyl-5-(triazol-1-yl)-pyrrolidindion(2.4)-Zink-chlorid

N-(2.3-Dichlorphenyl)-3.3-dimethyl-5-(imidazol-1-yl)-pyrrolidindion(2.4)-hydrochlorid

N-(2.3-Dichlorphenyl)-3.3-dimethyl-5-(imidazol-1-yl)-pyrrolidindion(2.4)-hydrogensulfat

N-(2.3-Dichlorphenyl)-3.3-dimethyl-5-(imidazol-1-yl)-pyrrolidindion(2.4)-cuprichlorid

N-(2.6-Dichlorphenyl)-3.3-dimethyl-5-(imidazol-1-yl)-pyrrolidindion(2.4)-hydrogennitrat

N-(2.6-Dichlorphenyl)-3.3-dimethyl-5-(imidazol-1-yl)-pyrrolidindion(2.4)-Zink-chlorid

N-(2.6-Dichlorphenyl)-3.3-dimethyl-5-(imidazol-1-yl)-pyrrolidindion(2.4)-tosylat

N-(4-Chlorphenyl)-3.3-dimethyl-4-oximino-5-(1.2.4-triazol-1-yl)-pyrrolidinon(2)

N-(2.4-Dichlorphenyl)-3.3-dimethyl-4-oximino-5-(1.2.4-triazol-1-yl)-pyrrolidinon(2)

N-(4-Bromphenyl)-3.3-dimethyl-4-oximino-5-(1.2.4-triazol-1-yl)-pyrrolidinon(2)

N-(4-Methoxyphenyl)-3.3-dimethyl-4-oximino-5-(1.2.4-triazol-1-yl)-pyrrolidinon(2)

N-(2-Methoxyphenyl)-3.3-dimethyl-4-oximino-5-(1.2.4-triazol-1-yl)-pyrrolidinon(2)

N-(3.5-Dichlorphenyl)-3.3-dimethyl-4-oximino-5-(1.2.4-triazol-1-yl)-pyrrolidinon(2)

N-(2-Methyl-4-chlor-phenyl)-3.3-dimethyl-4-oximino-5-(1.2.4-triazol-1-yl)-pyrrolidinon(2)

N-(4-Nitrophenyl)-3.3-dimethyl-4-oximino-5-(1.2.4-triazol-1-yl)-pyrrolidinon(2)

N-(4-Phenyl-phenyl)-3.3-dimethyl-4-oximino-5-(1.2.4-triazol-1-yl)-pyrrolidinon(2)

N-[4-(4'-Chlorphenoxy)phenyl]-3.3-dimethyl-4-oximino-5-(1.2.4-triazol-1-yl)-pyrrolidinon(2)

N-(4-Chlorphenyl)-3.3-dimethyl-4-hydroxy-5-(1.2.4-triazol-1-yl)-pyrrolidinon(2)

N-(2.4-Dichlorphenyl)-3.3-dimethyl-4-hydroxy-5-(1.2.4-triazol-1-yl)-pyrrolidinon(2)

N-(4-Fluorphenyl)-3.3-dimethyl-4-oximino-5-(1.2.4-triazol-1-yl)-pyrrolidinon(2)

N-(4-Phenylphenyl)-3.3-dimethyl-5-(1.2.4-triazol-1-yl)-pyrrolidindion(2.4)

N-(4-Methylphenyl)-3.3-dimethyl-4-oximino-5-(1.2.4-triazol-1-yl)-pyrrolidinon(2)

N-(4-Phenoxyphenyl)-3.3-dimethyl-4-oximino-5-(1.2.4-triazol-1-yl)-pyrrolidinon(2)

N-(2.4-Dibromphenyl)-3.3-dimethyl-5-(1.2.4-triazol-1-yl)-pyrrolidindion(2.4)

N-(2.4-Dimethylphenyl)-3.3-dimethyl-5-(1.2.4-triazol-1-yl)-pyrrolidindion(2.4)

N-(2-Chlor-4-methylphenyl)-3.3-dimethyl-5-(1.2.4-triazol-1-yl)-pyrrolidindion(2.4)

N-(2-Fluor-4-chlorphenyl)-3.3-dimethyl-5-(1.2.4-triazol-1-yl)-pyrrolidindion(2.4)

N-(2-Chlor-4-methyl-phenyl)-3.3-dimethyl-4-oximino-5-(1.2.4-triazol-1-yl)-pyrrolidinon(2)

Die erfindungsgemäßen Verbindungen sind durch Umsetzen von 5-Halogen-pyrrolidin-2.4-dionen mit den entsprechenden Azolen oder Azolsalzen zugänglich.

Ein bevorzugtes Verfahren zur Herstellung von Verbindungen der allgemeinen Formel

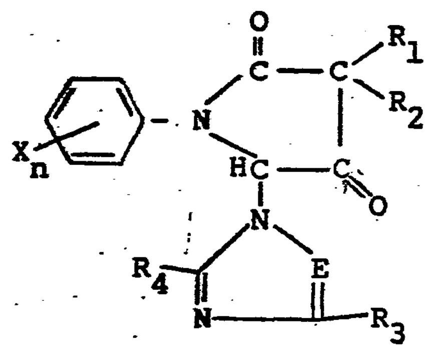

,

wobei

$R_1$ und $R_2$ gleiche oder verschiedene Alkylreste mit 1 bis 3 Kohlenstoffatomen bedeuten,

$R_3$ und $R_4$ gleiche oder verschiedene Substituenten sind mit der Bedeutung Wasserstoff, Alkyl mit 1 bis 4 Kohlenstoffatomen und Halogen,

E ein Stickstoffatom oder die ⋝CH-Gruppe ist

X für Substituenten steht, die ausgewählt sind aus der Gruppe der Halogene, der Alkylgruppen mit 1 bis 3 Kohlenstoffatomen, der substituierten Alkylgruppen mit 1 bis 3 Kohlenstoffatomen, der Cycloalkylgruppen mit 3 bis 6 Kohlenstoffatomen, der Alkoxygruppen mit 1 bis 3 Kohlenstoffatomen, der Alkylthiogruppen

mit 1 bis 3 Kohlenstoffatomen, der Alkylsulfonylgruppen mit 1 bis 3 Kohlenstoffatomen, der Acyloxygruppen mit 1 bis 3 Kohlenstoffatomen, der, ggf. substituierten, Phenyl- und Phenoxygruppen, der Cyano- und der Nitrogruppen,

und n 0 oder eine ganze Zahl von 1 bis 5 ist,

ist dadurch gekennzeichnet, daß Pyrrolidin-2.4-dione der Formel

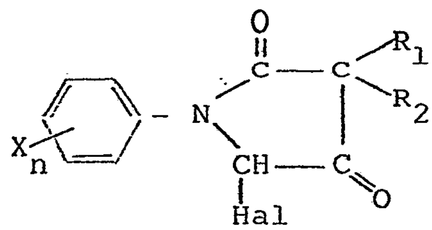

wobei

Hal für Cl oder Br steht und

$R_1$, $R_2$, X und n die oben angegebene Bedeutung haben

mit Azolen der Formel

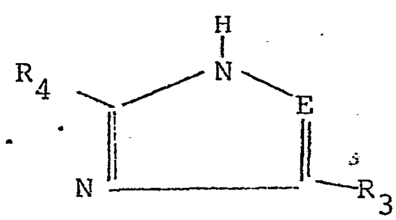

wobei

E, $R_3$ und $R_4$ die obige Bedeutung besitzen,

ggf. in Gegenwart von Säurebindemittel umgesetzt werden.

Als Säurebindemittel werden anorganische und/oder organische Basen eingesetzt.

Beispiele für anorganische Basen sind Alkali- und Erdalkalihydroxide, wie Natriumhydroxid, Kaliumhydroxid, Kalziumhydroxid; Alkali- und Erdalkalicarbonate, wie Natrium-, Kalium- oder Kalziumcarbonat.

Beispiele für organische Basen sind tertiäre Amine, wie Trimethylamin, Triethylamin, N.N-Dimethylanilin, N.N-Dimethylcyclohexylamin, Pyridin u.a.

Zumeist wird in polaren Lösungsmitteln gearbeitet. Beispiele hierfür sind Aceton, Tetrahydrofuran, Acetonitril u.a.

Die Reaktionstemperaturen liegen vorzugsweise im Bereich von 20 bis 80 °C.

Alternativ zu dem beschriebenen Herstellungsverfahren kann die Azol-Komponente auch in Form ihrer Alkali- und/oder Erdalkalisalze eingesetzt werden.

Dieses Verfahren zur Herstellung der Verbindungen der Formel

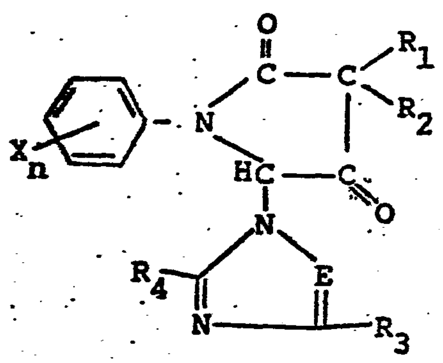

wobei

$R_1$, $R_2$, $R_3$, $R_4$, E, X und n die gleiche Bedeutung besitzen, wie in der bereits beschriebenen Verfahrensvariante angegeben,

ist dadurch gekennzeichnet, daß Pyrrolidin-2.4-dione der Formel

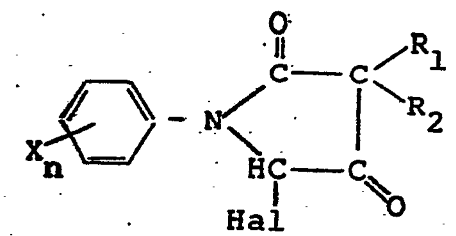

mit Azolen der Formel

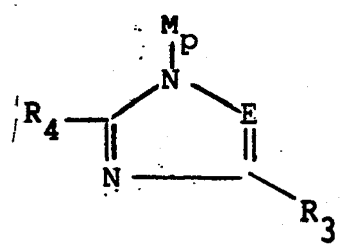

wobei

M für ein Alkali- oder Erdalkalimetall steht und
p die Werte 1/2 oder 1 annimmt

ggf. in Gegenwart von Phasentransferkatalysator umgesetzt werden.

Beispiele für M sind insbesondere Natrium, Kalium, Kalzium und Magnesium. p nimmt für Alkalimetalle den Wert 1, für Erdalkalimetalle den Wert 1/2 an.

Beispiele für Phasentransferkatalysatoren sind quartäre Ammoniumsalze, wie Tetrabutylammoniumjodid und Kronenether.

Zumeist wird in polaren Lösungsmitteln gearbeitet, wie Aceton, Acetonitril, Tetrahydrofuran und dergleichen.

Die Reaktionstemperaturen liegen vorzugsweise im Bereich von 20 bis 80 °C.

Typischerweise erfolgt die Aufarbeitung des Reaktionsgemisches bei beiden beschriebenen Verfahrensvarianten durch Abdestillieren des Lösungsmittels, Aufnehmen des Rückstandes in einem 2-Phasengemisch von Wasser/Methylenchlorid oder Wasser/Chloroform, wobei das Zielprodukt von wasserlöslichen Verunreinigungen befreit wird und schließlich durch Abdestillieren des organischen Lösungsmittels. Die Zielprodukte fallen zumeist als Öle an, die beim Stehenlassen auskristallisieren. Ggf. schließt sich noch ein weiterer Reinigungsschritt an, wie beispielsweise Umkristallisieren oder Behandeln des Produkts mit Ether.

Erfindungsgemäße Verbindungen, bei denen A für = CH - OH steht, sind in an sich bekannter Weise durch Reduktion der entsprechenden Ketoverbindung zugänglich.

Ein geeignetes Reduktionsverfahren ist katalytische Hydrierung des Ketons mit molekularem Wasserstoff in Gegenwart von Ruthenium- oder Platin-Trägerkatalysatoren. Als Trägermaterial kommen Aktivkohle und oxidische Spezien des Aluminiums und/oder des Siliciums in Betracht.

Weiterhin können zur Reduktion der Keto-Funktion komplexe Hydride, wie Natriumborhydrid oder Lithiumaluminiumhydrid, eingesetzt werden.

Die erfindungsgemäßen Oxim-azolyl-pyrrolidinone, bei denen A für die = C = N - OH steht, sind in an sich bekannter Weise durch Umsetzen der entsprechenden Ketoverbindung mit Hydroxylamin zugänglich.

Typischerweise wird Hydroxylamin in Salzform, z.B. in Form des Hydrochlorids, eingesetzt. Die Umsetzungen werden in Lösungsmitteln vorgenommen, wie beispielsweise Ethylalkohol oder wäßrig-alkoholischer Lösung, wobei Reaktionstemperaturen von 20 bis 100 °C, insbesondere 50 bis 80 °C, eingehalten werden. Ggf. werden noch Säurebindemittel zugesetzt, wie beispielsweise Natriumacetat.

Die Isolierung und Reindarstellung der Oxim-azolyl-pyrrolidinone erfolgt beispielsweise durch Ausfällen des Oxims mit Wasser aus der alkoholischen Lösung oder durch Abdestillieren des Lösungsmittels, Aufnehmen des Rückstandes in einem 2-Phasengemisch von Wasser und z.B. Essigsäureethylester, nachfolgender Entfernung des Lösungsmittels und ggf. weiterer Reinigung durch Umkristallisieren.

Die erfindungsgemäßen Oxim-azolyl-pyrrolidinone können sowohl in ihrer "Syn"- bzw. "Anti"-Form vorliegen. Im Rahmen der Erfindung liegen sowohl die beiden genannten Isomere sowie die Isomerengemische, die üblicherweise bei der Synthese anfallen.

Die Säureadditionssalze der erfindungsgemäßen Verbindungen lassen sich darstellen durch die Formel

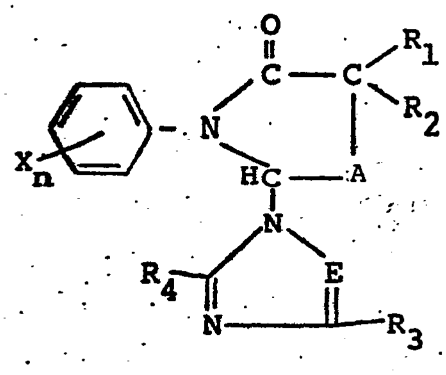

$\cdot \frac{1}{m} \ (H_m Y)$

Erfindungsgemäß wird eine bevorzugte Auswahl unter den Säureadditionssalzen unter dem Gesichtspunkt ihrer physiologischen Verträglichkeit getroffen.

m ist 1 für einbasige, 2 für zweibasige Säuren usw.

Die Bedeutung für Y ergibt sich aus der nachfolgenden beispielhaften Aufzählung von in Frage kommenden Säuren für die Säureadditionssalze:

Salzsäure, Bromwasserstoffsäure, Phosphorsäure, Salpetersäure, Schwefelsäure, mono- und bifunktionelle Carbonsäuren und Hydroxycarbonsäuren, wie Essigsäure, Maleinsäure, Bernsteinsäure, Salicylsäure sowie Sulfonsäuren, wie Toluolsulfonsäure.

Die Herstellung der Säureadditionssalze kann nach den auch bereits bisher bekannten Salzbildungsmethoden erfolgen, z.B. durch Lösen einer Verbindung der Formel

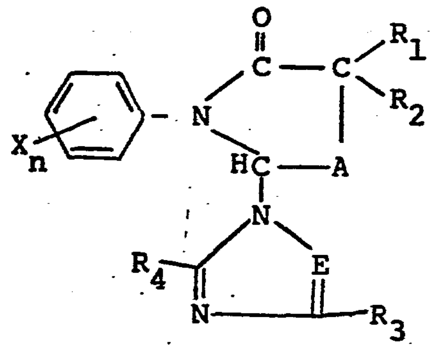

in einem polaren organischen Lösungsmittel und Hinzufügen der Säure in stöchiometrischen Mengen.

Die Isolierung des Zielprodukts erfolgt dann zweckmäßigerweise durch Abdampfen des Lösungsmittels im Vakuum.

Beispiele für derartige Lösungsmittel sind Diethylether, Acetonitril und insbesondere Alkohole, wie Ethylalkohole sowie deren wäßrige Lösungen.

Die erfindungsgemäßen Metallsalzkomplexe sind den Säureadditionssalzen analoge Verbindungen, bei denen der Säurewasserstoff durch ein Metallkation ersetzt ist. Beispiele für derartige Kationen sind solche, die sich von den Metallen der zweiten bis vierten Hauptgruppe, der ersten und zweiten sowie der achten Nebengruppe des Periodensystems ableiten, wie Magnesium, Kalzium, Zinn, Kupfer, Zink, Eisen und Nickel. Die entsprechenden Anionen leiten sich vorzugsweise ab von den Halogenwasserstoffsäuren sowie der Phosphor-, der Salpeter- und der Schwefelsäure. Ihre stöchiometrische Zusammensetzung ist analog den oben beschriebenen Säureadditionssalzen, wobei die Pyrrolidinon-Komponente stets einwertig ist.

Die Herstellung der Metallsalzkomplexe erfolgt ebenfalls analog den Additionssäuresalzen. Typischerweise wird eine äquimolare Menge des Metallsalzes in wäßrigem Ethanol gelöst und das entsprechende erfindungsgemäße Pyrrolidinon, zumeist gelöst in Ethanol, zugetropft. Schließlich wird das Zielprodukt in praktisch quantitativer Ausbeute durch Abdestillieren des Lösungsmittelgemisches im Vakuum erhalten.

Die zur Synthese der erfindungsgemäßen Azolylpyrrolidinone als Ausgangsverbindungen einzusetzenden 3.3-Dialkyl-pyrrolidin(2.4-dione) der Formel

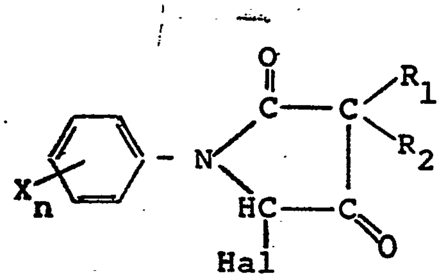

sind noch nicht bekannt. Sie können durch die üblichen Halogenierungsmethoden, beispielsweise Photobromierung, Umsetzungen mit Halogensuccinimiden oder Umsetzung mit Sulfurylchlorid aus den 3.3-Dialkylpyrrolidin-(2.4-dionen

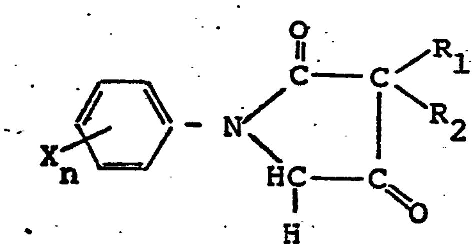

hergestellt werden. Die 3.3-Dialkylpyrrolidin-(2.4)-dione sind durch Ringschlußreaktion der offenkettigen Anilide der Formel

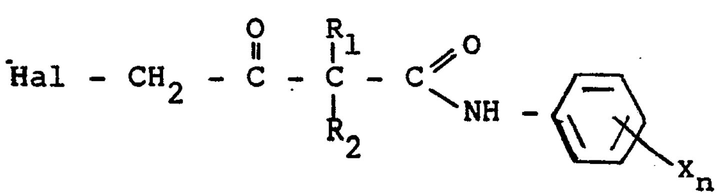

zugänglich. Diese Ringschlußreaktionen werden in Gegenwart von in Bezug auf freigesetzte Säure äquimolaren Mengen an tertiären Aminen, wie Triethylamin, Dimethylcyclohexylamin, Pyridin und dergl. durchgeführt. Die Reaktionstemperaturen liegen bevorzugt im Bereich von 90 bis 120 °C.

Die obengenannten Anilide sind wiederum aus den entsprechenden Säurehalogeniden in an sich bekannter Weise durch Umsetzung der entsprechenden Säurehalogenide mit, ggf. substituiertem, Anilin zugänglich.

Die genannten Säurehalogenide werden durch die allgemeine Formel

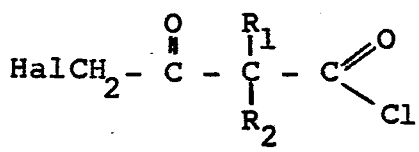

wiedergegeben.

Hal steht für Chlorid oder Bromid, insbesondere für Chlorid.

Für den Fall, daß $R_1$ und $R_2$ jeweils Methyl bedeuten, sind diese Verbindungen durch die an sich bekannte Einschubreaktion von Dimethylketen mit Halogenessigsäurechlorid zugänglich.

Die entsprechenden höheren Homologen, bei denen mindestens einer der Reste $R_1$ oder $R_2$ für Ethyl und/oder Propyl stehen, können in an sich bekannter Weise über die Alkylierung von Acetessigsäureester mit Ethyl- bzw. Propylhalogenid mit anschließender Chlorierungsreaktion gewonnen werden.

Die erfindungsgemäßen Verbindungen weisen fungitoxische Eigenschaften auf. Sie bewähren sich bei der Bekämpfung pilzlicher Krankheiten. Dabei entfalten sie eine besonders gute Wirksamkeit gegen Mehltauarten, wie beispielsweise Erysiphe graminis, Erysiphe cichoriacearum, Podosphaera leucotricha, Sphaerotheca pannosa, Sphaerotheca mors-uvae usw. Weitere Wirkungsbeispiele sind Schadpilze, wie Fusarium nivale, Tilletia caries, Helminthosporium gramineum, Uromyces phaseoli und Puccinia-Arten.

Die erfindungsgemäßen fungiziden Wirkstoffe wirken sowohl systemisch wie auch als Kontaktfungizide. Sie können sowohl protektiv, als auch kurativ eingesetzt werden. Besonders hervorzuheben ist die Tatsache, daß mit den erfindungsgemäßen Wirkstoffen auch solche Pilzstämme bekämpft werden können, die gegenüber den bekannten Benzimidazol-Fungiziden resistent geworden sind.

Sie eignen sich, ohne daß ihr Anwendungsbereich etwa darauf beschränkt wäre, z.B. zum Einsatz im Getreideanbau, im Gartenbau, insbesondere in Gurken- und Kürbispflanzungen oder bei Zierpflanzen sowie im Obstanbau. Als weitere erfindungsgemäße Anwendung wurde der Einsatz als Saatgutbeizmittel gefunden.

Die erfindungsgemäßen Wirkstoffe können allein oder im Gemisch mit anderen pestizid-, insbesondere fungizid-wirksamen Mitteln ausgebracht werden.

Im allgemeinen werden sie als Mischungen mit festen oder flüssigen Verdünnungsmitteln oder als Lösungen in festen oder flüssigen Lösungsmitteln verwendet, mit Wirkstoffgehalten von 0,01 bis 95 Gew.%.

Die Mischungen bzw. Lösungen werden im allgemeinen als Emulsionskonzentrate, Suspensionskonzentrate, Pasten, Spritzpulver, Stäubemittel, Granulate oder Mikrokapseln hergestellt.

Emulsionskonzentrate und Pasten enthalten im allgemeinen 10 bis 60 Gew.%, vorzugsweise 15 bis 40 Gew.%, Wirkstoff, 2 bis 25 Gew.% Dispergierhilfsstoffe und organische Lösungsmittel und/oder Wasser.

Spritzpulver enthalten meist 10 bis 80 Gew.%, vorzugsweise 15 bis 70 Gew.%, Wirkstoff, 1 bis 10 Gew.% Dispergierhilfsstoffe und 10 bis 89 Gew.% inerte Bestandteile.

Granulate und Stäubemittel enthalten neben inerten Bestandteilen, Bindemitteln und/oder Überzugsstoffen 1 bis 10 Gew.%, vorzugsweise 5 bis 10 Gew.%, Wirkstoff. Erfindungsgemäß angewandt werden:

Als Dispergierhilfsstoffe z.B. Alkyl- und Arylsulfonate, Methylzellulose, polymere Sulfonsäuren und deren Salze, Polyalkohole, Fettsäureester, Fettalkoholether, Fettamine;

als organische Lösungsmittel z.B. Alkohole, wie Ethanol, Butanole, Dimethylformamid, Dimethylsulfoxid, N-Methylpyrrolidon, Aromaten, wie Toluol und Xylole;

als inerte Bestandteile z.B. Kaolin, China-Clay, Talkum, Kalziumcarbonat, hochdisperse Kieselsäure, Kieselgele, Kieselgur, Diatomenerde, Bims, Maisschrot, Verdickungsmittel, wie Stärke und Carboxymethylzellulose;

als Bindemittel z.B. Magnesiumsulfat, Gips, Gummiarabikum.

Beispielsweise werden die erfindungsgemäßen Fungizide wie folgt formuliert:

1. Emulsionskonzentrat:

   50 Gew.% Wirkstoff
   42 Gew.% N-Methyl-pyrrolidon
   8 Gew.% Nonylphenolpolyglykolether

2. Spritzpulver:

   50 Gew.% Wirkstoff
   41,5 Gew.% Kieselerde (Handelsname "Silitin")
   2 Gew.% Natriumligninsulfonat
   5 Gew.% Alkylpolyglykolether
   1,5 Gew.% Polypropylenglykol

3. Suspensionskonzentrat:

50 Gew.% Wirkstoff
5 Gew.% Ethylenglykol
1,5 Gew.% hochdisperse Kieselsäure ("HDK")
0,5 Gew.% Entschäumungsmittel
5 Gew.% Octylphenolpolyglykolether
38 Gew.% Wasser

Die Aufwandmengen an Wirkstoff können in großen Bereichen variieren. Bei der Saatgutbehandlung werden im allgemeinen Wirkstoffmengen von 0,05 bis 25 g/kg Saatgut benötigt.

Die Ausbringung der erfindungsgemäßen Wirkstoffe kann in jeder geeigneten Form erfolgen. Beispielhaft genannt seien:

Gießen, Verspritzen, Versprühen, Verstäuben, Bestreichen, Behandeln des Saatguts (Beizen).

Die Erfindung wird nun anhand von Beispielen näher erläutert:

<u>Beispiel 1</u>

Herstellung von N-Phenyl-3.3-dimethyl-5-(1.2.4-triazol-1-yl)-pyrrolidindion(2.4)

a) 239,5 g 4-Chloro-3-oxo-2.2-dimethylbutancarbonsäureanilid (1 Mol) werden mit 101 g Triethylamin in 2 l Toluol 3 Stunden auf 110 °C erhitzt. Danach wird das Reaktionsgemisch mit verdünnter Salzsäure und anschließend mit Wasser gewaschen. Nach dem Abziehen des Toluols verbleibt ein öliger Rückstand, der in der Kälte auskristallisiert. Das kristalline Produkt wird noch durch Behandeln mit wenig n-Hexan/Ether-Gemisch gereinigt.

Es wurden 185 g, entsprechend 91 % der Theorie, an N-Phenyl-3.3-dimethyl-pyrrolidindion(2.4) erhalten.

b) 203 g N-Phenyl-3.3-dimethyl-pyrrolidindion(2.4) (1 Mol) werden in 2 l Chlorbenzol auf 100 °C erhitzt und im Verlauf von 6 Stunden 162 g Sulfurylchlorid (1,2 Mol) und 5 g Azobisisobutyronitril (gelöst in 30 ml Chlorbenzol) zugetropft. Nach dem Abdestillieren des Chlorbenzols im Vakuum verbleibt ein öliger Rückstand, der in der Kälte auskristallisiert. Das kristalline Produkt wird noch durch Behandeln mit wenig n-Hexan/Ether-Gemisch gereinigt.

Es wurden 171 g, entsprechend 72 % der Theorie, an N-Phenyl-3.3-dimethyl-5-chloro-pyrrolidindion(2.4) erhalten.

c) 23,7 g N-Phenyl-3.3-dimethyl-5-chloro-pyrrolidindion(2.4) (0,1 Mol) wurden in 250 ml Aceton gelöst, mit 7,6 g 1.2.4-Triazol(0,11 Mol) und 13,8 g wasserfreiem Kaliumcarbonat versetzt und unter Rühren 6 Stunden lang unter Rückfluß erhitzt. Nach Abkühlen des Reaktionsgemisches wurde Abfiltriert und das Lösungsmittel im Vakuum abgezogen. Der Rückstand wurde in Chloroform gelöst und die Lösung mit 2-n-Salzsäure und anschließend mit Wasser gewaschen. Nach dem Abdestillieren des Chloroforms im Vakuum verblieb ein kristalliner Rückstand, der zur Reinigung noch mit wenig Diethylether behandelt wurde.

Es wurde in einer Ausbeute von 24,7 g, entsprechend 91,5 % der Theorie, an Zielprodukt erhalten.

Analysendaten:

| | | | |
|---|---|---|---|
| ber.: | C 62,21 %, | H 5,22 %, | N 20,73 % |
| gef.: | C 62,3 %, | H 5,2 %, | N 20,8 % |

<u>Beispiel 2</u>

Herstellung von N-(4-Chlorphenyl)-3.3-dimethyl-5-(1.2.4-triazol-1-yl)-pyrrolidindion(2.4)

27,2 g N-(4-Chlorphenyl)-3.3-dimethyl-5-chloro-pyrrolidindion(2.4) (0,1 Mol), hergestellt analog Beispiel 1 a) und b), wurden zusammen mit 9,1 g Natrium-1.2.4-triazol (0,1 Mol)

in 250 ml Acetonitril unter Rühren 2 Stunden auf 80 °C erhitzt. Nach dem Abkühlen des Reaktionsgemisches wurde das Lösungsmittel im Vakuum abdestilliert und der Rückstand in Methylenchlorid aufgenommen. Diese Lösung wurde nun mit Wasser gewaschen und schließlich das Methylenchlorid im Vakuum wieder entfernt. Es hinterblieb ein Öl, das nach einiger Zeit auskristallisierte.

Das Zielprodukt wurde in einer Ausbeute von 27,1 g, entsprechend 98 % der Theorie, erhalten.

Analysendaten:

| | | | | |
|---|---|---|---|---|
| ber.: | C 55,18 %, | H 4,30 %, | Cl 11,63 %, | N 18,39 % |
| gef.: | C 55,1 %, | H 4,2 %, | Cl 11,0 %, | N 18,9 % |

<u>Beispiel 3</u>

Herstellung von N-(4-Chlorphenyl)-3.3-dimethyl-4-hydroxy-5-(1.2.4-triazol-1-yl)-pyrrolidinon(2)

15,2 g N-(4-Chlorphenyl)-3.3-dimethyl-5-(1.2.4-triazol-1-yl)-pyrrolidindion(2.4) (0,05 Mol) gemäß Beispiel 2 wurden in 250 ml Isopropanol gelöst und bei 20 °C unter Rühren portionsweise mit 2,7 g Natriumborhydrid (0,07 Mol) versetzt. Das Reaktionsgemisch wurde noch 20 Stunden bei 20 °C gerührt. Danach wurden 200 ml Wasser zugesetzt und weitere 8 Stunden gerührt. Nach Abdestillieren des Isopropanol-Wasser-Gemisches im Vakuum wurde der Rückstand mit 6 n-Salzsäure auf pH 5 gebracht. Danach wurde mit Chloroform mehrmals extrahiert und die wäßrigen Phasen verworfen. Nach Entfernen des Chloroforms im Vakuum verblieb ein kristalliner Feststoff, der zur Reinigung aus Ethanol umkristallisiert wurde.

Das Zielprodukt fiel in einer Ausbeute von 12,7 g, entsprechend 23 % der Theorie, an.

Analysendaten:

| | | | | |
|---|---|---|---|---|
| ber.: | C 54,81 %, | H 4,93 %, | Cl 11,56 %, | N 18,26 % |
| gef.: | C 54,3 %, | H 5,0 %, | Cl 11,1 % | N 18,7 % |

Beispiel 4

Herstellung von N-(4-Chlorphenyl)-3.3-dimethyl-4-oximino-5-(1.2.4-triazol-1-yl)-pyrrolidinon(2)

19,8 g N-(4-Chlorphenyl)-3.3-dimethyl-5-(1.2.4-triazol-1-yl)-pyrrolidindion (2.4) (0,065 Mol), gemäß Beispiel 2, sowie 9,0 g Hydroxylammoniumhydrochlorid (0,13 Mol) und 14,9 g Natriumacetat (0,18 Mol) wurden in 150 ml Ethanol unter Rühren 1 Stunde auf Rückfluß erhitzt. Nach dem Abkühlen des Reaktionsgemisches wurde abfiltriert und anschließend das Ethanol im Vakuum entfernt. Der Rückstand wurde noch mit Diethylether, anschließend mit Wasser, gewaschen und schließlich im Vakuum getrocknet.

Es wurden 18,3 g, entsprechend 88 % der Theorie, an Zielprodukt erhalten.

Analysendaten:

| | | | | |
|---|---|---|---|---|
| ber. | C 52,59 %, | H 4,41 %, | Cl 11,09 %, | N 21,9 % |
| gef. | C 52,8 %, | H 4,2 %, | Cl 11,0 %, | N 22,1 % |

Beispiel 5

Herstellung von N-(2.4-Dichlorphenyl)-3.3-dimethyl-5-(imidazol-1-yl)-pyrrolidindion(2.4)

30,6 g N-(2.4-Dichlorphenyl)-3.3-dimethyl-5-chloro-pyrrolidindion(2.4) (0,1 Mol) und 9 g Natriumimidazol in 250 ml Acetonitril wurden unter Rühren 1/2 Stunde auf 60 °C erwärmt. Nach dem Abkühlen des Reaktionsgemisches wurde das Lösungsmittel im Vakuum abgezogen. Der Rückstand wurde mit Methylenchlorid/Wasser ausgeschüttelt und die wäßrige Phase verworfen. Nach Entfernen des Methylenchlorids im Vakuum aus der organischen Phase verblieb ein kristalliner Feststoff, der noch mit wenig Diethylether gereinigt wurde.

Es wurden 30,4 g, entsprechend 90 % der Theorie, an Zielprodukt erhalten.

Analysendaten:

| | | | | |
|---|---|---|---|---|
| ber.: | C 53,27 %, | H 3,87 %, | Cl 20,97 %, | N 12,43 % |
| gef.: | C 53,4 %, | H 3,9 %, | Cl 21,0 %, | N 12,7 % |

Beispiel 6

Herstellung von N-(4-Chlorphenyl)-3.3-dimethyl-5-(1.2.4-triazol-1-yl)-pyrrolidindion(2.4)-hydrochlorid

0,05 Mol N-(4-Chlorphenyl)-3.3-dimethyl-5-(1.2.4-triazol-1-yl)-pyrrolidindion(2.4) wurden in 200 ml Ethanol gelöst. Dieser Lösung wurden unter Rühren 5 ml 10 n-Salzsäure zugetropft. Danach wurde das Alkohol/Wasser-Gemisch im Vakuum abdestilliert und das zurückbleibende Zielprodukt im Vakuum getrocknet.

Ausbeute 98 % der Theorie. Schmelzpunkt 115 °C.

Beispiel 7

Prophylaktische Wirkung gegen Erysiphe cichoriacearum (Spritztest)

Bis zum 2-Blatt-Stadium angezogene Gurkenpflanzen wurden nach Entfernen der unter den Laubblättern befindlichen Kotyledonen mit als Spritzbrühen formulierten erfindungsgemäßen Wirkstoffen bis zur Tropfnässe bespritzt. Nach Antrocknen des Spritzbelags erfolgte die Beimpfung der Pflanzen durch gleichmäßiges Bestäuben der Blattoberflächen mit Konidien des Pilzes Erysiphe cichoriacearum. Die Pflanzen wurden anschließend bei 22 bis 24 °C und einer relativen Luftfeuchtigkeit von 70 bis 80 % im Gewächshaus aufgestellt.

Spätestens 15 Tage nach der künstlichen Infektion bzw. nach 100 %-igem Befall unbehandelter Kontrollpflanzen erfolgte die Bonitur.

Der Wirkungsgrad bei entsprechender Aufwandmenge wurde im Vergleich zu unbehandelten Kontrollpflanzen nach der Formel

$$100 - \frac{\text{befallene Blattfläche, behandelt}}{\text{befallene Blattfläche, unbehandelt}} \times 100$$

ermittelt.

Es wurden die folgen erfindungsgemäßen Wirkstoffe eingesetzt:

A = N-(2.4-Dichlorphenyl)-3.3-dimethyl-5-(1.2.4-triazol-1-yl)-pyrrolidindion(2.4)-hydrochlorid
B = N-(2.4-Dichlorphenyl)-3.3-dimethyl-5-(imidazol-1-yl)-pyrrolidindion(2.4)
C = N-(4-Chlorphenyl)-3.3-dimethyl-5-(imidazol-1-yl)-pyrrolidindion(2.4)-hydrochlorid
D = N-(2.4-Dichlorphenyl)-3.3-dimethyl-5-(1.2.4-triazol-1-yl)-pyrrolidindion(2.4)

Tabelle 1

Wirksamkeit erfindungsgemäßer Aktivsubstanzen als Kontaktfungizide in % gegen Erysiphe cichoriacearum

| | W i r k s t o f f | | | |
|---|---|---|---|---|
| ppm Aktivsubstanz | A | B | C | D |
| 60 | 100 | 100 | 100 | 100 |
| 30 | 100 | 95 | 100 | 90 |
| 16 | 100 | 90 | 100 | - |
| 8 | 100 | - | 80 | - |

Beispiel 8

Prophylaktische Wirkung erfindungsgemäßer Wirkstoffe gegen Erysiphe cichoracearum durch Gießbehandlung

Es wurde die systemische Wirkung erfindungsgemäßer Aktivsubstanzen getestet.

In Plastiktöpfen (Inhalt 180 $cm^3$ Erdsubstrat) angezogene Gurkenpflanzen der Sorte "chinesische Schlangen" wurden im 2-Blatt-Stadium nach Entfernen der unter den Laubblättern befindlichen Kotyledonen mit pro Topf 50 ml Spritzbrühe, deren Wirkstoffgehalt in Tabelle 2 angegeben ist, angegossen. 24 Stunden nach Bodenapplikation erfolgte die Beimpfung der Blätter mit Konidien des Pilzes Erysiphe cichoriacearum.

Die Weiterbehandlung und Bonitur der Pflanzen erfolgte wie in Beispiel 7 beschrieben.

Es wurden die bereits in Beispiel 7 definierten Wirkstoffe A, B, C, ferner

E = N-(4-Chlorphenyl)-3.3-dimethyl-4-oximo-5-(1.2.4-triazol-1-yl)-pyrrolidinon(2)

F = N-(4-Chlorphenyl)-3.3-dimethyl-5-(1.2.4-triazol-1-yl)-pyrrolidindion(2.4)

Tabelle 2

Systemische Wirksamkeit erfindungsgemäßer Aktivsubstanzen in % gegen Erysiphe cichoriacearum

| ppm Aktivsubstanz | Wirkstoff A | B | C | D | E | F |
|---|---|---|---|---|---|---|
| 60 | 100 | 100 | 90 | 100 | 100 | 100 |
| 30 | 95 | 90 | - | 100 | 100 | 95 |
| 16 | 85 | - | - | 90 | 100 | - |

Vergleichsbeispiel 1

Es wurde die Arbeitsweise gemäß den Beispielen 7 und 8 wiederholt, mit der Abänderung, daß als Aktivsubstanzen die Wirkstoffe Pyrazophos und Triforine eingesetzt wurden.

Ergebnisse:

Tabelle 3

Fungizide Wirksamkeit der Vergleichssubstanzen in % gegen Erysiphe cichoriacearum

| Wirkstoff | Spritztest | | | Gießbehandlung | | |
|---|---|---|---|---|---|---|
| | Aufwandmenge in ppm | | | | | |
| | 60 | 30 | 15 | 60 | 30 | 15 |
| Pyrazophos | 50 | - | - | 50 | 40 | - |
| Triforine | 80 | 70 | 60 | 0 | 0 | 0 |

Beispiel 9

Kurative Wirkung erfindungsgemäßer Aktivstoffe gegen Erysiphe graminis sp. hordei

Gerstenpflanzen einer mehltauanfälligen Sorte wurden in Plastiktöpfen angezogen und nach Erreichen des 3-Blatt-Stadiums mit Konidien des Mehltaupilzes Erysiphe graminis sp. hordei inokuliert. Danach erfolgte die Aufstellung im Gewächshaus bei Temperaturen von 20 °C und einer relativen Luftfeuchte von 70 bis 80 %.

3 Tage nach der ersten Beimpfung mit dem Pilzerreger wurden die Pflanzen mit erfindungsgemäßen Wirkstoffen in den entsprechenden Konzentrationen tropfnaß gespritzt.

Über den Versuchspflanzen angeordnete Ventilatoren sorgten auch nach der primären Inokulierung fortlaufend für eine ständige Neuinfektion der Pflanzen mit Pilzsporen, die von stark befallenen, gleichmäßig verteilten Gerstenpflanzen stammten.

Nach einer Inkubationszeit von 14 Tagen wurden die Pflanzen auf Befall mit Erysiphe graminis sp. hordei bonitiert, wobei eine unbehandelte Probe als Vergleich diente.

Der Befallsgrad wurde ausgedrückt in % befallener Blattfläche, bezogen auf unbehandelte, infizierte Kontrollpflanzen (= 100 % Befall).

Als Aktivstoffe wurden die in den Beispielen 7 und 8 mit D und F bezeichneten Wirkstoffe

eingesetzt.

Tabelle 4

Kurative Wirkung gegen Erysiphe graminis mittels Blattspritzung

| | befallene Blattfläche in % | | |
|---|---|---|---|
| | Aufwandmenge in ppm | | |
| Wirkstoff | 125 | 60 | 30 |
| D | 0 | 5 | 20 |
| F | 10 | 20 | - |

Vergleichsbeispiel 2

Es wurde die Arbeitsweise gemäß Beispiel 9 wiederholt, mit der Abänderung, daß als Aktivsubstanzen die handelsüblichen Wirkstoffe Tridemorph und Benomyl eingesetzt wurden.

Ergebnisse:

Tabelle 5

Kurative Wirkung gegen Erysiphe graminis mittels Blattspritzung

| | befallene Blattfläche in % | | |
|---|---|---|---|
| | Aufwandmenge in ppm | | |
| Wirkstoff | 125 | 60 | 30 |
| Tridemorph | 60 | - | - |
| Benomyl | 90 | 100 | - |

Beispiel 10

Kurative Wirkung gegen Erysiphe graminis sp. hordei durch Gießbehandlung

Es wurden Gerstenpflanzen herangezogen und mit Konidien des Mehltaupilzes Erysiphe graminis sp. hordei inokuliert wie in Beispiel 8 beschrieben. 3 Tage nach dem primären Inokulieren erfolgte die Gießbehandlung mit jeweils 50 ml Spritzbrühe pro Topf. Bei fortlaufender Infektion (wie in Beispiel 9 beschrieben) wurde schließlich nach einer Inkubationszeit von 14 Tagen die Auswertung auf befallene Blätter vorgenommen.

Folgende erfindungsgemäßen Wirkstoffe wurden eingesetzt: B, D, E, F (definiert in den Beispielen 7 und 8) und

H = N-(2.4-Dichlorphenyl)-3.3-dimethyl-5-(1.2.4-triazol-1-yl)-pyrrolidindion(2.4)-hydrogensulfat

Tabelle 6

Kurative Wirkung gegen Erysiphe graminis sp. hordei durch Gießbehandlung

| Wirkstoff | befallene Blattfläche in % Aufwandmenge in ppm | | | |
|---|---|---|---|---|
| | 60 | 30 | 16 | 8 |
| B | 0 | 5 | 15 | - |
| D | 0 | 0 | 5 | 10 |
| E | 0 | 0 | 0 | 5 |
| F | 0 | 5 | 15 | - |
| H | 0 | 5 | 15 | - |

Vergleichsbeispiel 3

Es wurde die Arbeitsweise gemäß Beispiel 9 wiederholt, mit der Abänderung, daß die handelsüblichen Wirkstoffe Tridemorph und Benomyl eingesetzt wurden.

Ergebnisse:

Tabelle 7

| Wirkstoff | befallene Blattfläche in % Aufwandmenge in ppm | | | |
|---|---|---|---|---|
| | 60 | 30 | 16 | 8 |
| Benomyl | 60 | 75 | - | - |
| Tridemorph | 100 | 100 | - | - |

Beispiel 11

Kurative Wirkung gegen Sphaerotheca pannosa

Es wurde die kurative Wirkung gegen Sphaerotheca pannosa unter Freilandbedingungen getestet.

An unbehandelten Freilandrosen der Sorte "Superstar" konnte sich unter den vorherrschenden Witterungsbedingungen ein natürlicher Befall an echtem Mehltau (Sphaerotheca pannosa) aufbauen. Vom Pilz waren alle oberirdischen Pflanzenteile befallen, besonders stark aber die Blätter der Rosenstöcke.

Die Behandlung der befallenen Rosenstöcke begann, als ca. 25 % der Blätter vom Myzel des Pilzes bedeckt waren.

Während der Versuchsdauer herrschte überwiegend freundliches Sommerwetter mit wenig Niederschlag, Temperaturen um 25 °C und einer relativen Luftfeuchte von 70 %.

Als erfindungsgemäßer Wirkstoff wurde N-(2.4-Dichlorphenyl)-3.3-dimethyl-5-(1.2.4-triazol-1-yl)-pyrrolidindion(2.4) (Wirkstoff D) eingesetzt, der als Spritzbrühe mit einer

Aktivsubstanzkonzentration von 250 ppm formuliert war. Bei jeder Applikation wurden die oberirdischen Pflanzenteile der Rosenstöcke bis zur Tropfnässe gleichmäßig bespritzt. Es erfolgten 3 Applikationen, wie in der Praxis üblich, im Abstand von 3 Tagen und 2 weitere im Abstand von jeweils 10 Tagen.

Als Vergleich und Kontrolle dienten unbehandelte Rosenstöcke sowie solche, die in der gleichen Aktivsubstanzkonzentration und in der gleichen Weise mit dem Standardwirkstoff Triforine behandelt wurden.

Die Bonitur auf Befall erfolgte in Zeitabständen wie in der folgenden Tabelle angegeben:

Tabelle 8

| | mit Sphaerotheca pannosa befallene Blattfläche in % | | |
|---|---|---|---|
| Anzahl der Tage ab Versuchsbeginn | behandelt mit Wirkstoff D | behandelt mit Triforine | unbehandelt |
| 0 | 25 | 25 | 25 |
| 11 | 15 | 20 | 35 |
| 32 | 0 | 5 | 50 |
| 59 | 0 | 5 | 70 |

Die Ergebnisse gemäß Tabelle 8 demonstrieren den Bekämpfungserfolg des erfindungsgemäßen Wirkstoffs anhand der zügig eintretenden Verminderung und schließlichen Beseitigung des Befalls.

## Patentansprüche

1. Verbindungen der allgemeinen Formel

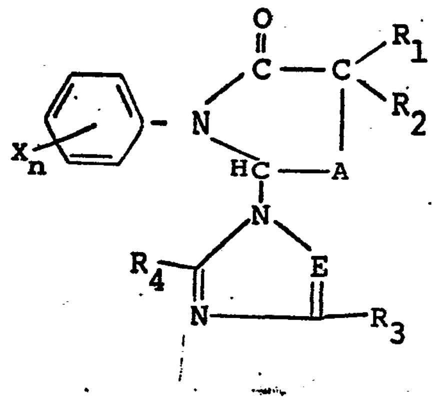

sowie deren Säureadditionssalze und Metallsalz-Komplexe, wobei

$R_1$ und $R_2$ gleiche oder verschiedene Alkylreste mit 1 bis 3 Kohlenstoffatomen bedeuten,

$R_3$ und $R_4$ gleiche oder verschiedene Substituenten sind mit der Bedeutung Wasserstoff, Alkyl mit 1 bis 4 Kohlenstoffatomen und Halogen,

A für eine Ketogruppe, für ein funktionelles Derivat einer Ketogruppe und für eine alkoholische Gruppe der Formel = CH - OH steht,

E ein Stickstoffatom oder die ⪢CH-Gruppe bedeutet,

X für Substituenten steht, die ausgewählt sind aus der Gruppe der Halogene, der Cyano- und der Nitrogruppen, der Alkylgruppen mit 1 bis 3 Kohlenstoffatomen, der substituierten Alkylgruppen mit 1 bis 3 Kohlenstoffatomen, der Cycloalkylgruppen mit 3 bis 6 Kohlenstoffatomen, der Alkoxygruppen mit 1 bis 3 Kohlenstoffatomen, der Alkylthiogruppen mit 1 bis 3 Kohlenstoffatomen, der Alkylsulfonylgruppen mit 1 bis 3 Kohlenstoffatomen, der Acyloxygruppen mit 1 bis 3 Kohlenstoffatomen und der, ggf. substituierten Phenyl- und Phenoxygruppen und

n 0 oder eine ganze Zahl von 1 bis 5 ist.

2. Verbindungen nach Anspruch 1, bei denen $R_1$ und $R_2$ Methylgruppen bedeuten.

3. Fungizid wirksame Zusammensetzungen, die mindestens eine Verbindung nach den Ansprüchen 1 und 2 enthalten.

4. Verfahren zur Herstellung von Verbindungen der allgemeinen Formel

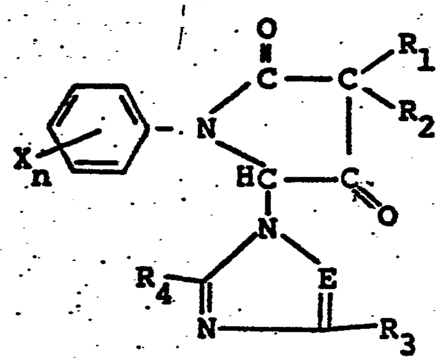

wobei

$R_1$ und $R_2$ gleiche oder verschiedene Alkylreste mit 1 bis 3 Kohlenstoffatomen bedeuten,

$R_3$ und $R_4$ gleiche oder verschiedene Substituenten sind mit der Bedeutung Wasserstoff, Alkyl mit 1 bis 4 Kohlenstoffatomen und Halogen,

E ein Stickstoffatom oder die ⩾CH-Gruppe ist,

X für Substituenten steht, die ausgewählt sind aus der Gruppe der Halogene, der Alkylgruppen mit 1 bis 3 Kohlenstoffatomen, der substituierten Alkylgruppen mit 1 bis 3 Kohlenstoffatomen, der Cycloalkylgruppen mit 3 bis 6 Kohlenstoffatomen, der Alkoxygruppen mit 1 bis 3 Kohlenstoffatomen, der Alkylthiogruppen mit 1 bis 3 Kohlenstoffatomen, der Alkylsulfonylgruppen mit 1 bis 3 Kohlenstoffatomen, der Acyloxygruppen mit 1 bis 3 Kohlenstoffatomen, der,

ggf. substituierten, Phenyl- und Phenoxygruppen, der Cyano- und der Nitrogruppen,

und n 0 oder eine ganze Zahl von 1 bis 5 ist,

ist dadurch gekennzeichnet, daß Pyrrolidin-2.4-dione der Formel

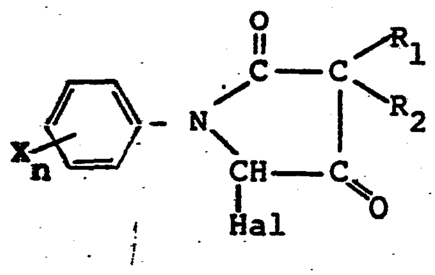

wobei

Hal für Cl und Br steht und

$R_1$ und $R_2$ die oben angegebene Bedeutung haben

mit Azolen der Formel

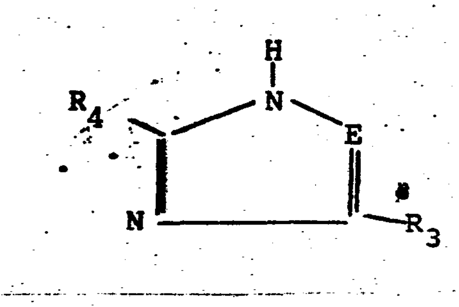

wobei

E, $R_3$ und $R_4$ die obige Bedeutung besitzen,

ggf. in Gegenwart von Säurebindemittel umgesetzt werden.